# EUROPÄISCHE PATENTANMELDUNG

(11) **EP 1 172 362 A1**
(43) Veröffentlichungstag der Anmeldung: **16.01.2002**
(21) Anmeldenummer: 01115716.1
(22) Anmeldetag: 06.07.2001
(51) Int. Cl.: C07D 273/00, C07D 413/12, C07D 417/12, A01N 43/88, A61K 31/539

(54) **Azadioxacycloalkene und ihre Verwendung zur Bekämpfung von Schadpilzen und tierischen Schädlingen**

(30) Priorität: 11.07.2000 DE 10033509
(71) Anmelder: BASF AKTIENGESELLSCHAFT, 67056 Ludwigshafen (DE)
(72) Erfinder: Gewehr, Markus, 56288 Kastellaun (DE); Sauter, Hubert, 68167 Mannheim (DE); Gypser, Andreas, 68159 Mannheim (DE); Grammenos, Wassilios, 67063 Ludwigshafen (DE); Tormo I Blasco, Jordi, 67117 Limburgerhof (DE); Müller, Bernd, 67227 Frankenthal (DE); Cullmann, Oliver Dr., 64646 Heppenheim (DE); Stierl, Reinhard, 67112 Mutterstadt (DE); Ammermann, Eberhard, 64646 Heppenheim (DE); Strathmann, Siegfried, 67117 Limburgerhof (DE); Lorenz, Gisela, 67434 Neustadt (DE)
(74) Vertreter: Fitzner, Uwe, Dr.

(57) **Zusammenfassung**

Die Erfindung betrifft Azadioxacycloalkene der Formel I, 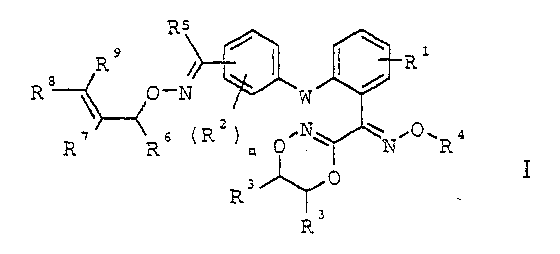 in der W -OCH₂-, -C(R¹⁰)=N-O-CH₂- bedeutet, und die Subtituenten R¹ bis R¹⁰ sowie der Index n die in der Beschreibung angegebene Bedeutung haben.

Die erfindungsgemäßen Verbindungen sind zur Bekämpfung von Schadpilzen und tierischen Schädlingen brauchbar.

## Beschreibung

Die vorliegende Erfindung betrifft Azadioxacycloalkene der Formel I, 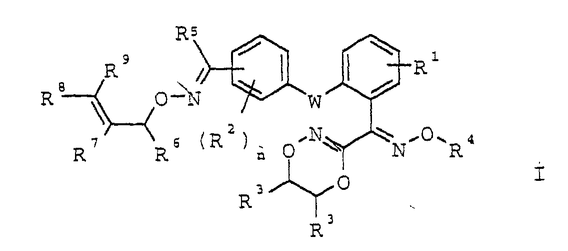 in der die Substituenten R¹ bis R¹⁰, das Brückenglied W und der Index n die folgenden Bedeutungen haben:
- W: -OCH₂-, -C(R¹⁰)=N-O-CH₂- ;
- R¹: Wasserstoff, C₁-C₄-Alkyl, Halogen, Nitro, CN, C₁-C₄-Halogenalkyl, C₁-C₄-Alkoxy;
- R²: Wasserstoff, C₁-C₄-Alkyl, Halogen, Nitro, CN, C₁-C₄-Halogenalkyl, C₁-C₄-Alkoxy;
- n: 1 oder 2;
- R³: Wasserstoff, Methyl oder Ethyl;
- R⁴: C₁-C₄-Alkyl;
- R⁵: Wasserstoff, C₁-C₆-Alkyl, C₂-C₄-Alkenyl, C₁-C₄-Halogenalkyl, unsubstituiertes oder durch Halogen, C₁-C₄-Alkyl, C₁-C₄-Halogenalkyl, C₁-C₄-Alkoxy oder C₁-C₄-Halogenalkoxy substituiertes Phenyl, C₃-C₆-Cycloalkyl;
- R⁶: Wasserstoff, C₁-C₄-Alkyl, C₁-C₄-Halogenalkyl, C₂-C₄-Alkenyl, unsubstituiertes oder durch Halogen, C₁-C₄-Alkyl, C₁-C₄-Halogenalkyl, C₁-C₄-Alkoxy oder C₁-C₄-Halogenalkoxy substituiertes Phenyl;
- R⁷: Wasserstoff, Halogen, C₁-C₆-Alkyl, C₁-C₄-Halogenalkyl, C₂-C₆-Alkenyl, C₂-C₆-Halogenalkenyl, C₃-C₆-Cycloalkyl, C₃-C₆-Halogencycloalkyl, unsubstituiertes oder durch Halogen, C₁-C₄-Alkyl, C₁-C₄-Halogenalkyl, C₁-C₄-Alkoxy oder C₁-C₄-Halogenalkoxy substituiertes Phenyl;
- R⁸: Wasserstoff, Halogen, C₁-C₆-Alkyl, C₁-C₆-Halogenalkyl, C₂-C₆-Alkenyl, C₂-C₆-Halogenalkenyl, C₃-C₆-Cycloalkyl, C₃-C₆-Halogencycloalkyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl, oder
- R⁷ und R⁸: bilden, zusammen mit den Kohlenstoffatomen, an die sie gebunden sind, einen ungesättigten Heterocyclus mit 5- oder 6-Ringatomen, der ein oder zwei Heteroatome aufweist, die unabhängig voneinander ausgewählt sind unter einem Stickstoff-, Sauerstoff- und Schwefelatom und der gegebenenfalls mit einem oder zwei Resten substituiert sein kann, die unabhängig voneinander ausgewählt sind unter C₁-C₄-Alkyl, Halogen, Nitro, CN, C₁-C₄-Halogenalkyl, C₁-C₄-Alkoxy, C₂-C₄-Alkenyl, C₂-C₄-Halogenalkenyl, C₂-C₄-Alkinyl, C₂-C₄-Halogenalkinyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl;
- R⁹: Wasserstoff, Halogen, C₁-C₆-Alkyl, C₁-C₆-Halogenalkyl, C₂-C₆-Alkenyl, C₂-C₆-Halogenalkenyl, C₃-C₆-Cycloalkyl, C₃-C₆-Halogencycloalkyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl;
- R¹⁰: Wasserstoff, Halogen, C₁-C₄-Alkyl.

Weiterhin betrifft die Erfindung Mittel zur Bekämpfung von Schadpilzen und tierischen Schädlingen, welche die Verbindungen der Formel I enthalten, und die Verwendung der Verbindungen I zur Bekämpfung von pflanzenpathogenen Pilzen und tierischen Schädlingen.

Fungizid wirkende Azadioxacycloalkene sind bereits bekannt. Beispielsweise werden in WO-A 95/04728, WO-A 97/00866 und WO-A 97/27189 Strobilurine mit einem Azadioxacycloalken-Pharmakophor beschrieben. Die bekannten Wirkstoffe weisen am zentralen Benzolring, in ortho-Stellung zum Pharmakophor, unter anderem eine (Het)aryloxy-Gruppe oder eine α-Bisoximether-Gruppierung auf.

Desweiteren sind Fungizide aus der Klasse der Strobilurine bekannt, die eine ungesättigte Oximetherfunktion in der Seitenkette tragen. In der EP-A 597124 und EP-A 673923 sind Wirkstoffe mit einer Phenoxymethyl-Seitenkette, wobei der Phenylrest eine Alkenyloxyiminoalkyl-Gruppe aufweist, beschrieben.

Davon ausgehend, liegt der vorliegenden Erfindung die Aufgabe zugrunde, Verbindungen mit verbesserter Wirkung und/oder verbreitertem Wirkungsspektrum bereitzustellen. Überraschenderweise wurde nun gefunden, dass diese Aufgabe gelöst wird mit Verbindungen, welche einen Azadioxacycloalken-Pharmakophor und eine ungesättigte Oximether-Seitenkette aufweisen.

Die vorstehend aufgeführten Bedeutungen stellen Sammelbegriffe für individuelle Aufzählungen der einzelnen Gruppenmitglieder dar. Sämtliche Kohlenstoffketten können geradkettig oder verzweigt sein. Halogenierte Substituenten tragen vorzugsweise 1 bis 5 gleiche oder verschiedene Halogenatome.

Im Einzelnen bedeuten beispielsweise:
- Halogen: Fluor, Chlor, Brom, Jod, vorzugsweise Fluor oder Chlor;
- C₁-C₄-Alkyl: Methyl, Ethyl, n-Propyl, 1-Methylethyl, n-Butyl, 1-Methylpropyl, 2-Methylpropyl, 1,1-Dimethylethyl, vorzugsweise Methyl;
- C₁-C₆-Alkyl: C₁-C₄-Alkyl wie vorstehend genannt, sowie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, vorzugsweise Methyl, Ethyl, n-Propyl, 1-Methylethyl, n-Butyl oder 1,1-Dimethylethyl;
- C₂-C₆-Alkenyl: Ethenyl, Prop-1-en-1-yl, Prop-2-en-1-yl, 1-Methylethenyl, n-Buten-1-yl, n-Buten-2-yl, n-Buten-3-yl, l-Methylprop-l-en-l-yl, 2-Methylprop-l-en-l-yl, 1-Methyl-prop-2-en-1-yl, 2-Methylprop-2-en-1-yl, n-Penten-1-yl, n-Penten-2-yl, n-Penten-3-yl, n-Penten-4-yl, 1-Methylbut-1-en-1-yl, 2-Methylbut-l-en-l-yl, 3-Methylbut-l-en-l-yl, l-Methylbut-2-en-1-yl, 2-Methylbut-2-en-1-yl, 3-Methylbut-2-en-1-yl, 1-Methylbut-3-en-1-yl, 2-Methylbut-3-en-1-yl, 3-Methylbut-3-en-1-yl, 1,1-Dimethyl-prop-2-en-1-yl, 1,2-Dimethyl-prop-1-en-l-yl, 1,2-Dimethyl-prop-2-en-1-yl, 1-Ethylprop-1-en-2-yl, 1-Ethylprop-2-en-1-yl, n-Hex-1-en-1-yl, n-Hex-2-en-1-yl, n-Hex-3-en-1-yl, n-Hex-4-en-1-yl, n-Hex-5-en-1-yl, l-Methylpent-l-en-l-yl, 2-Methyl-pent-1-en-1-yl, 3-Methylpent-1-en-1-yl, 4-Methylpent-1-en-1-yl, l-Methylpent-2-en-1-yl, 2-Methylpent-2-en-1-yl, 3-Methylpent-2-en-1-yl, 4-Methylpent-2-en-1-yl, 1-Methylpent-3-en-1-yl, 2-Methylpent-3-en-1-yl, 3-Methylpent-3-en-l-yl, 4-Methylpent-3-en-1-yl, 1-Methylpent-4-en-1-yl, 2-Methylpent-4-en-1-yl, 3-Methylpent-4-en-1-yl, 4-Methylpent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethylbut-1-en-1-yl, 1-Ethylbut-2-en-1-yl, 1-Ethylbut-3-en-1-yl, 2-Ethylbut-1-en-1-yl, 2-Ethylbut-2-en-1-yl, 2-Ethylbut-3-en-1-yl, 1,1,2-Trimethylprop-2-en-1-yl, 1-Ethyl-1-methyl-prop-2-en-1-yl, 1-Ethyl-2-methyl-prop-1-en-1-yl, 1-Ethyl-2-methyl-prop-2-en-1-yl, vorzugsweise Ethenyl oder Prop-2-en-1-yl;
- C₂-C₄-Alkinyl: Ethinyl, Prop-1-in-1-yl, Prop-2-in-3-yl, n-But-1-in-1-yl, n-But-1-in-4-yl, n-But-2-in-1-yl, vorzugsweise Prop-2-in-1-yl;
- C₁-C₂-Halogenalkyl: z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, vorzugsweise Difluormethyl oder Trifluormethyl;
- C₁-C₆-Halogenalkyl: C₁-C₆-Alkyl wie vorstehend genannt, das partiell oder vollständig durch Fluor, Chlor und/oder Brom substituiert ist, also z. B. die vorstehend genannten C₁-C₂-Halogenalkylreste, sowie 3-Chlorpropyl oder Heptafluorpropyl, vorzugsweise Trifluormethyl, Pentafluorethyl oder Heptafluorpropyl;
- C₂-C₆-Halogenalkenyl: C₂-C₆-Alkenyl wie vorstehend genannt, das partiell oder vollständig durch Fluor, Chlor und/oder Brom substituiert ist, also z. B. 2-Chlorallyl, 3-Chlorallyl oder 3,3-Dichlorallyl;
- C₂-C₄-Halogenalkinyl: C₂-C₄-Alkinyl wie vorstehend genannt, das partiell oder vollständig durch Fluor, Chlor und/oder Brom substituiert ist, z. B. Chlorethinyl, 3-Chlorpropinyl;
- C₃-C₆-Cycloalkyl: Cyclopropyl, Cyclobutyl, Cyclopentyl, Cyclohexyl, vorzugsweise Cyclopropyl, Cylopentyl oder Cyclohexyl;
- C₃-C₆-Halogencycloalkyl: C₃-C₆-Cycloalkyl wie vorstehend genannt, das partiell oder vollständig durch Fluor, Chlor und/ oder Brom substituiert ist, also z. B. 2-, 3- oder 4-Chlorcyclopentyl, 2-, 3- oder 4-Chlorcyclohexyl, 2,3,4-Trichlorcyclopentyl oder 2,3,4,5,6,-Pentachlorcyclohexyl;

Der ungesättigte Heterocyclus mit 5- oder 6-Ringatomen kann aromatisch oder nicht-aromatisch sein. Bei einem aromatischen Heterocyclus handelt es sich insbesondere um 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, insbesondere Furanyl, Thienyl, Oxazolyl und Thiazolyl.

Wenn der Phenylrest substituiert ist, weist er vorzugsweise einen oder zwei Substituenten auf, die unabhängig voneinander ausgewählt sind unter Halogen, insbesondere Fluor oder Chlor, C₁-C₄-Alkyl, C₁-C₄-Halogenalkyl, C₁-C₄-Alkoxy, Nitro und CN. Bevorzugt ist Halogen und/oder C₁-C₄-Alkyl.

Vorzugsweise haben die Substituenten in der Formel 1 folgende Bedeutung:
- R¹: Wasserstoff, C₁-C₄-Alkyl, Halogen, C₁-C₄-Halogenalkyl, insbesondere Wasserstoff oder C₁-C₄-Alkyl;
- R²: Wasserstoff, C₁-C₄-Alkyl, Halogen, Halogen-C₁-C₄-Alkyl, insbesondere Wasserstoff oder C₁-C₄-Alkyl;
- R³: Wasserstoff;
- R⁴: Methyl oder Ethyl und insbesondere Methyl;
- R⁵: Wasserstoff oder C₁-C₄-Alkyl;
- R⁶: Wasserstoff, C₁-C₄-Alkyl oder C₁-C₄-Halogenalkyl, insbesondere Wasserstoff oder C₁-C₄-Alkyl;
- R⁷: Wasserstoff, Halogen, C₁-C₆-Alkyl, Halogen-C₁-C₆-Alkyl, C₃-C₆-Cycloalkyl, C₃-C₆-Halogencycloalkyl oder Phenyl, insbesondere Wasserstoff, Halogen, C₁-C₆-Alkyl und besonders bevorzugt Wasserstoff oder Halogen;
- R⁸: Wasserstoff, Halogen, C₁-C₆-Alkyl, Halogen-C₁-C₆-Alkyl, C₃-C₆-Cycloalkyl, C₃-C₆-Halogencycloalkyl, C₂-C₆-Alkenyl oder Phenyl, insbesondere Wasserstoff, Halogen, C₁-C₆-Alkyl; oder
- R⁷ und R⁸: stehen zusammen, mit den Kohlenstoffatomen, an die sie gebunden sind, für Thienyl, Furyl, Oxazolyl oder Thiazolyl, wobei diese Gruppen gegebenenfalls substituiert sind durch C₁-C₄-Alkyl, Halogen oder Phenyl, das gegebenenfalls durch ein oder zwei Halogen substituiert sein kann und insbesondere für Thienyl oder Oxazolyl, wobei diese Gruppen gegebenenfalls durch ein oder zwei Halogen oder Phenyl substituiert sein können und der Phenyl-Substituent seinerseits durch ein oder zwei Halogen substituiert sein kann;
- R⁹: Wasserstoff, Halogen, C₁-C₆-Alkyl, C₁-C₆-Halogenalkyl, C₃-C₆-Cycloalkyl, C₃-C₆-Halogencycloalkyl oder Phenyl, insbesondere Wasserstoff, Halogen, C₁-C₆-Alkyl und besonders bevorzugt Wasserstoff oder Halogen;
- R¹⁰: Wasserstoff oder C₁-C₄-Alkyl und insbesondere Wasserstoff oder Methyl.

Die Oxim-Seitenkette kann in o-, m- oder p-Position zu W an den Phenylring gebunden sein. Bevorzugt ist die p-Position.

Der Rest R¹ steht vorzugsweise in 6-Position.

Wenn die Oxim-Seitenkette in p-Position zu W gebunden ist, steht der Rest R2 vorzugsweise in 2- und/oder 5-Position.

Bevorzugte Ausführungsformen sind die Verbindungen der Formel I, worin die Substituenten die folgenden Bedeutungen haben:
A)
   - W: -OCH₂-, -C(R¹⁰)=N-O-CH₂;
   - R¹: Wasserstoff, C₁-C₄-Alkyl, Halogen, C₁-C₄-Halogenalkyl;
   - R²: Wasserstoff, C₁-C₄-Alkyl, Halogen, C₁-C₄-Halogenalkyl;
   - R³: Wasserstoff, Methyl oder Ethyl;
   - n: 1 oder 2;
   - R⁴: Methyl oder Ethyl;
   - R⁵: Wasserstoff oder C₁-C₄-Alkyl;
   - R⁶: Wasserstoff, C₁-C₄-Alkyl, C₁-C₄-Halogenalkyl;
   - R⁷: Wasserstoff, Halogen, C₁-C₆-Alkyl, C₁-C₆-Halogenalkyl, C₃-C₆-Cycloalkyl, C₃-C₆-Halogencycloalkyl, Phenyl;
   - R⁸: Wasserstoff, Halogen, C₁-C₆-Alkyl, C₁-C₆-Halogenalkyl, C₃-C₆-Cycloalkyl, C₃-C₆-Halogencycloalkyl, C₂-C₆-Alkenyl, Phenyl, das durch ein oder zwei Halogen oder C₁-C₄-Alkyl substituiert sein kann; oder
   - R⁷ und R⁸: zusammen mit den Kohlenstoffatomen, an die sie gebunden sind, bilden einen ungesättigten Heterocyclus mit 5- oder 6-Ringatomen, der ein oder zwei Heteroatome aufweist, die unabhängig voneinander ausgewählt sind unter einem Stickstoff-, Sauerstoff- und Schwefelatom und der gegebenenfalls mit einem oder zwei Resten substituiert sein kann, die unabhängig voneinander ausgewählt sind unter C₁-C₄-Alkyl, Halogen, Halogen-C₁-C₄-Alkyl, C₁-C₄-Alkoxy und Phenyl, das gegebenenfalls durch ein oder zwei Halogen oder C₁-C₄-Alkyl substituiert ist;
   - R⁹: Wasserstoff, Halogen, C₁-C₆-Alkyl, C₁-C₆-Halogenalkyl, C₃-C₆-Cycloalkyl, C₃-C₆-Halogencycloalkyl, Phenyl;
   - R¹⁰: Wasserstoff, Halogen, C₁-C₄-Alkyl.
B)
   - W: -OCH₂-, -C(R₁₀)=N-O-CH₂;
   - R¹: Wasserstoff, Methyl, Halogen;
   - R²: Wasserstoff, C₁-C₄-Alkyl, Halogen;
   - n: 1 oder 2;
   - R³: Wasserstoff;
   - R⁴: Methyl oder Ethyl;
   - R⁵: Wasserstoff, C₁-C₄-Alkyl;
   - R⁶: Wasserstoff, C₁-C₄-Alkyl;
   - R⁷: Wasserstoff, Halogen, C₁-C₆-Alkyl;
   - R⁸: Wasserstoff, Halogen, C₁-C₆-Alkyl, C₂-C₆-Alkenyl; oder
   - R⁷ und R⁸: bilden zusammen mit den Kohlenstoffatomen, an die sie gebunden sind, Thienyl, Furanyl, Oxazolyl, Thiazolyl, wobei diese Gruppen ein oder zwei Substituenten aufweisen können, die unabhängig voneinander ausgewählt sind unter C₁-C₄-Alkyl, Halogen und Phenyl, das gegebenenfalls durch ein oder zwei Halogen substituiert ist;
   - R⁹: Wasserstoff, Halogen, C₁-C₆-Alkyl;
   - R¹⁰: Wasserstoff, C₁-C₄-Alkyl;
C)
   - W: -OCH₂-, -C(R₁₀)=N-O-CH₂;
   - R¹: Wasserstoff;
   - R²: Wasserstoff, C₁-C₄-Alkyl, Halogen;
   - n: 1 oder 2;
   - R³: Wasserstoff;
   - R⁴: Methyl oder Ethyl;
   - R⁵: Wasserstoff, C₁-C₄-Alkyl;
   - R⁶: Wasserstoff, C₁-C₄-Alkyl;
   - R⁷: Wasserstoff, Halogen;
   - R⁸: Wasserstoff, C₁-C₄-Alkyl, Halogen; oder
   - R⁷ und R⁸: bilden zusammen mit den Kohlenstoffatomen, an die sie gebunden sind Thienyl oder Oxazolyl, wobei diese Gruppen gegebenenfalls durch ein oder zwei Halogen oder Phenyl substituiert sind und das Phenyl gegebenenfalls durch ein oder zwei Halogen substituiert ist;
   - R⁹: Wasserstoff, Halogen;
   - R¹⁰: Wasserstoff, C₁-C₄-Alkyl.

   Weitere Ausführungsformen sind
D) Verbindungen der Formeln Ia und Ib: 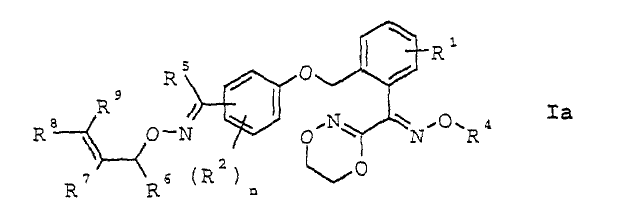 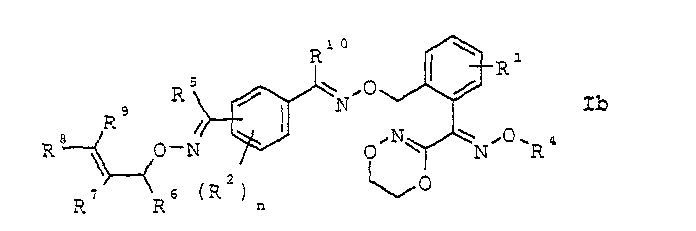
worin die Substituenten die folgende Bedeutungen besitzen:
- R¹: Wasserstoff, 6-Methyl oder 6-Chlor;
- R²: Wasserstoff, 2-Methyl, 5-Methyl, 2,5-Dimethyl, 2-Chlor ;
- n: 1 oder 2,
- R⁴: Methyl, Ethyl;
- R⁵: Wasserstoff, Methyl, Ethyl, n-Propyl, iso-Propyl, Cyclopropyl, Phenyl;
- R⁶: Wasserstoff, Methyl;
- R⁷: Wasserstoff, Methyl, Halogen;
- R⁸: Wasserstoff, C₁-C₄-Alkyl, Halogen, Phenyl;
- R⁹: Wasserstoff, Methyl, Chlor;
- R¹⁰: Wasserstoff, Methyl.

Insbesondere sind im Hinblick auf ihre Verwendung die in den folgenden Tabellen zusammengestellten Verbindungen der Formeln Ic bis If bevorzugt. Die dabei für einen Substituenten genannten Gruppen stellen außerdem für sich betrachtet, unabhängig von der Kombination, in der sie genannt sind, eine besonders bevorzugte Ausgestaltung des betreffenden Substituenten dar:

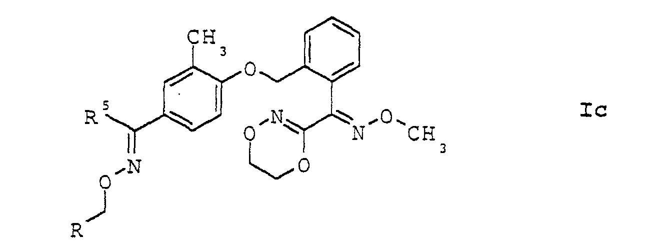

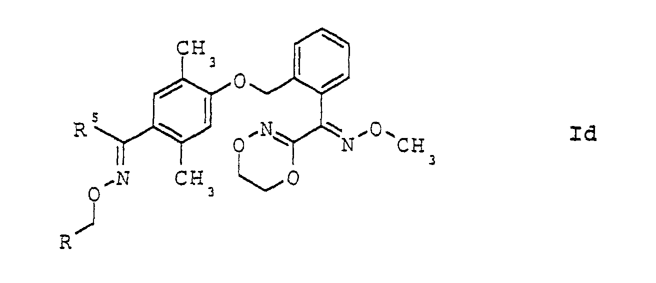

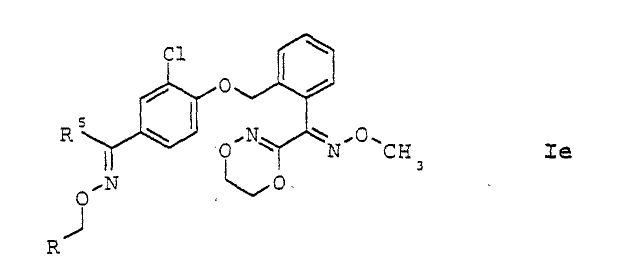

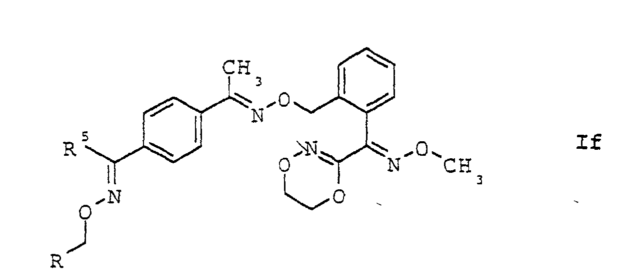

### Tabelle 1

Verbindungen der allgemeinen Formel Ic, in der R⁵ für Wasserstoff steht und R einer Zeile der Tabelle A entspricht.

### Tabelle 2

Verbindungen der allgemeinen Formel Id, in der R⁵ für Wasserstoff steht und R einer Zeile der Tabelle A entspricht.

### Tabelle 3

Verbindungen der allgemeinen Formel Ie, in der R⁵ für Wasserstoff steht und R einer Zeile der Tabelle A entspricht.

### Tabelle 4

Verbindungen der allgemeinen Formel If, in der R⁵ für Wasserstoff steht und R einer Zeile der Tabelle A entspricht.

### Tabelle 5

Verbindungen der allgemeinen Formel Ic, in der R⁵ für Methyl steht und R einer Zeile der Tabelle A entspricht.

### Tabelle 6

Verbindungen der allgemeinen Formel Id, in der R⁵ für Methyl steht und R einer Zeile der Tabelle A entspricht.

### Tabelle 7

Verbindungen der allgemeinen Formel Ie, in der R⁵ für Methyl steht und R einer Zeile der Tabelle A entspricht.

### Tabelle 8

Verbindungen der allgemeinen Formel If, in der R⁵ für Methyl steht und R einer Zeile der Tabelle A entspricht.

### Tabelle 9

Verbindungen der allgemeinen Formel Ic, in der R⁵ für Ethyl steht und R einer Zeile der Tabelle A entspricht.

### Tabelle 10

Verbindungen der allgemeinen Formel Id, in der R⁵ für Ethyl steht und R einer Zeile der Tabelle A entspricht.

### Tabelle 11

Verbindungen der allgemeinen Formel Ie, in der R⁵ für Ethyl steht und R einer Zeile der Tabelle A entspricht.

### Tabelle 12

Verbindungen der allgemeinen Formel If, in der R⁵ für Ethyl steht und R einer Zeile der Tabelle A entspricht.

### Tabelle 13

Verbindungen der allgemeinen Formel Ic, in der R⁵ für n-Propyl steht und R einer Zeile der Tabelle A entspricht.

### Tabelle 14

Verbindungen der allgemeinen Formel Id, in der R⁵ für n-Propyl steht und R einer Zeile der Tabelle A entspricht.

### Tabelle 15

Verbindungen der allgemeinen Formel Ie, in der R⁵ für n-Propyl steht und R einer Zeile der Tabelle A entspricht.

### Tabelle 16

Verbindungen der allgemeinen Formel If, in der R⁵ für n-Propyl steht und R einer Zeile der Tabelle A entspricht.

### Tabelle 17

Verbindungen der allgemeinen Formel Ic, in der R⁵ für i-Propyl steht und R einer Zeile der Tabelle A entspricht.

### Tabelle 18

Verbindungen der allgemeinen Formel Id, in der R⁵ für i-Propyl steht und R einer Zeile der Tabelle A entspricht.

### Tabelle 19

Verbindungen der allgemeinen Formel Ie, in der R⁵ für i-Propyl steht und R einer Zeile der Tabelle A entspricht.

### Tabelle 20

Verbindungen der allgemeinen Formel If, in der R⁵ für i-Propyl steht und R einer Zeile der Tabelle A entspricht.

### Tabelle 21

Verbindungen der allgemeinen Formel Ic, in der R⁵ für Cyclopropyl steht und R einer Zeile der Tabelle A entspricht.

### Tabelle 22

Verbindungen der allgemeinen Formel Id, in der R⁵ für Cyclopropyl steht und R einer Zeile der Tabelle A entspricht.

### Tabelle 23

Verbindungen der allgemeinen Formel Ie, in der R⁵ für Cyclopropyl steht und R einer Zeile der Tabelle A entspricht.

### Tabelle 24

Verbindungen der allgemeinen Formel If, in der R⁵ für Cyclopropyl steht und R einer Zeile der Tabelle A entspricht.

### Tabelle 25

Verbindungen der allgemeinen Formel Ic, in der R⁵ für Phenyl steht und R einer Zeile der Tabelle A entspricht.

### Tabelle 26

Verbindungen der allgemeinen Formel Id, in der R⁵ für Phenyl steht und R einer Zeile der Tabelle A entspricht.

### Tabelle 27

Verbindungen der allgemeinen Formel Ie, in der R⁵ für Phenyl steht und R einer Zeile der Tabelle A entspricht.

### Tabelle 28

Verbindungen der allgemeinen Formel If, in der R⁵ für Phenyl steht und R einer Zeile der Tabelle A entspricht.

### Tabelle A

Die Nennung von (E), (E,E) und (Z) bezieht sich auf die Substituenten an der in R angegebenen Doppelbindung.

| Nummer | R |
|---|---|
| 1. | -CH=CH₂ |
| 2. | (E) -CH=CH-CH₃ |
| 3. | (Z) -CH=CH-CH₃ |
| 4. | -CH=C(CH₃)₂ |
| 5. | (E) -CH=CH-C₂H₅ |
| 6. | (Z) -CH=CH-C₂H₅ |
| 7. | (E) -CH=C(CH₃)-C₂H₅ |
| 8. | (Z) -CH=C(CH₃)-C₂H₅ |
| 9. | -CH=C(C₂H₅)₂ |
| 10. | (E) -CH=CH-Cl |
| 11. | (Z) -CH=CH-Cl₃ |
| 12. | (E) -CH=C(Cl)-CH |
| 13. | (Z) -CH=C(Cl)-CH₃ |
| 14. | (E) -CH=C(Cl)-C₂H₅ |
| 15. | (Z) -CH=C(Cl)-C₂H₅ |
| 16. | -CH=CCl₂ |
| 17. | (E) -CH=CH-CH=CH₂ |
| 18. | (E,E) -CH=CH-CH=CH-CH₃ |
| 19. | (E,E) -CH=CH-CH=CH-C₆H₅ |
| 20. | (E, E) -CH=CH-CH=CH-(p-F-C₆H₄) |
| 21. | (E, E) -CH=CH-CH=CH-(p-Cl-C₆H₄) |
| 22. | -C(CH₃)=CH₂ |
| 23. | (E) -C(CH₃)=CH-CH₃ |
| 24. | (Z) -C(CH₃)=CH-CH₃ |
| 25. | -C(CH₃)=C(CH₃)₂ |
| 26. | (E) -C(CH₃)=CH-C₂H₅ |
| 27. | (Z) -C(CH₃)=CH-C₂H₅ |
| 28. | (E) -C(CH₃)=C(CH₃)-C₂H₅ |
| 29. | (Z) -C(CH₃)=C(CH₃)-C₂H₅ |
| 30. | -C(CH₃)=C(C₂H₅)₂ |
| 31. | (E) -C(CH₃)=CH-Cl |
| 32. | (Z) -C(CH₃)=CH-Cl |
| 33. | (E) -C(CH₃)=C(Cl)-CH₃ |
| 34. | (Z) -C(CH₃)=C(Cl)-CH₃ |
| 35. | (E) -C(CH₃)=C(Cl)-C₂H₅ |
| 36. | (Z) -C(CH₃)=C(Cl)-C₂H₅ |
| 37. | -C(CH₃)=CCl₂ |
| 38. | (E) -C(CH₃)=CH-CH=CH₂ |
| 39. | (E,E) -C(CH₃)=CH-CH=CH-CH₃ |
| 40. | (E, E) -C(CH₃)=CH-CH=CH-C₆H₅ |
| 41. | (E, E) -C(CH₃)=CH-CH-CH-(p-F-C₆H₄) |
| 42. | (E, E) -C(CH₃)=CH-CH=CH-(p-Cl-C₆H₄) |
| 43. | -C(C₂H₅)=CH₂ |
| 44. | (E) -C(C₂H₅)=CH-CH₃ |
| 45. | (Z) -C(C₂H₅)=CH-CH₃ |
| 46. | -C(C₂H₅)=C(CH₃)₂ |
| 47. | (E) -C(C₂H₅)=CH-C₂H₅ |
| 48. | (Z) -C(C₂H₅)=CH-C₂H₅ |
| 49. | (E) -C(C₂H₅)=C(CH₃)-C₂H₅ |
| 50. | (Z) -C(C₂H₅)=C(CH₃)-C₂H₅ |
| 51. | -C(C₂H₅)=C(C₂H₅)₂ |
| 52. | (E) -C(C₂H₅)=CH-Cl |
| 53. | (Z) -C(C₂H₅)=CH-Cl |
| 54. | (E) -C(C₂H₅)=C(Cl)-CH₃ |
| 55. | (Z) -C(C₂H₅)=C(Cl)-CH₃ |
| 56. | (E) -C(C₂H₅)=C(Cl)-C₂H₅ |
| 57. | (Z) -C(C₂H₅)=C(Cl)-C₂H₅ |
| 58. | -C(C₂H₅)=CCl₂ |
| 59. | (E) -C(C₂H₅)=CH-CH=CH₂ |
| 60. | (E,E) -C(C₂H₅)=CH-CH=CH-CH₃ |
| 61. | (E,E) -C(C₂H₅)=CH-CH=CH-C₆H₅ |
| 62. | (E,E) -C(C₂H₅)=CH-CH=CH-(p-F-C₆H₄) |
| 63. | (E,E) -C(C₂H₅)=CH-CH=CH-(p-Cl-C₆H₄) |
| 64. | Thiophen-2-yl |
| 65. | Thiophen-3-yl |
| 66. | Furan-2-yl |
| 67. | Furan-3-yl |
| 68. | 5-Chlor-thiophen-2-yl |
| 69. | 5-Brom-thiophen-2-yl |
| 70. | 5-Chlor-furan-2-yl |
| 71. | 5-Brom-furan-2-yl |
| 72. | 5-Methyl-thiophen-2-yl |
| 73. | 5-Phenyl-thiophen-2-yl |
| 74. | 5-Methyl-furan-2-yl |
| 75. | 5-Phenyl-furan-2-yl |
| 76. | Oxazol-4-yl |
| 77. | Oxazol-5-yl |
| 78. | Thiazol-4-yl |
| 79. | Thiazol-5-yl |
| 80. | 2-Phenyl-oxazol-4-yl |
| 81. | 2-Phenyl-thiazol-4-yl |
| 82. | 2-(p-Chlorphenyl-oxazol-4-yl |
| 83. | 2-(p-Chlorphenyl-thiazol-4-yl |
| 84. | 2-(p-Bromphenyl-oxazol-4-yl |
| 85. | 2-(p-Bromphenyl-thiazol-4-yl |
| 86. | 2-(p-Fluorphenyl-oxazol-4-yl |
| 87. | 2-(p-Fluorphenyl-thiazol-4-yl |
| 88. | 2-(2,4-Dichlorphenyl-oxazol-4-yl |
| 89. | 2-(2,4-Dichlorphenyl-thiazol-4-yl |

Die Verbindungen I können auf verschiedenen Wegen erhalten werden, wobei es für die Synthese unerheblich ist, ob zunächst A) der Azadioxacycloalken-Ring (s. Schemata 1 und 2) oder B) die orthoständige ungesättigte Oximether-Gruppierung aufgebaut wird.

A) Die für die Herstellung der Verbindungen I benötigten Verbindungen IV sind aus der Literatur bekannt [vgl. WO-A 95/04728; WO-A 98/17653]. Sie können beispielsweise auf dem folgenden Syntheseweg erhalten werden:

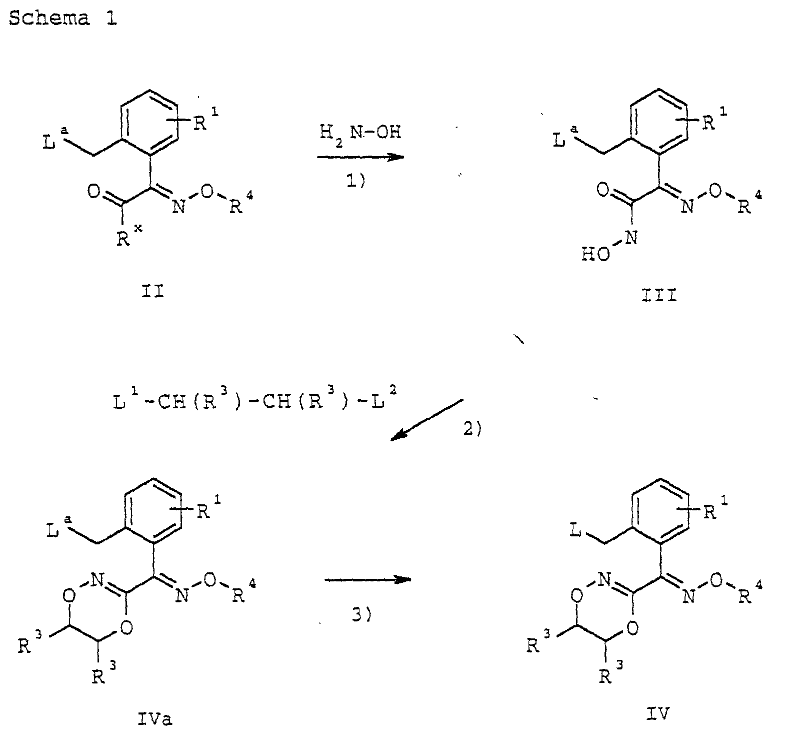

In den Formeln II, III und IVa steht L^{a} für ein durch eine Schutzgruppe geschütztes Sauerstoffatom; R^{x} in Formel II für Halogen, Alkylcarbonyloxy, OH, C₁-C₄-Alkoxy oder einen Aktivester wie gegebenenfalls substituiertes Phenoxy (wie beispielsweise p-Nitrophenoxy oder Pentafluorphenoxy), Succinimidoxy oder Isoharnstoff; R¹, R³ und R⁴ haben die zuvor genannte Bedeutung. Die Abgangsgruppe L in Formel IV sowie L¹ und L² in Formel L¹-[CH(R³)]₂-L² stehen jeweils für eine nucleophil austauschbare Gruppe wie Halogenid, C₁-C₄-Alkylsulfonat, C₁-C₁₂-Alkylphenylsulfonat oder Mono-C₁-C₄-alkylsulfat oder L¹ und L² können gemeinsam auch eine Brücke -O-darstellen. Im Folgenden werden die Schritte 1) bis 3) dieser Umsetzung näher erläutert.
1) Benzylverbindungen der Formel II werden mit Hydroxylamin oder dessen Säureadditionssalz gegebenenfalls in Gegenwart einer Base oder eines wasserentziehenden Mittels, wie N,N'-Dicyclohexylcarbodiimid, oder in Gegenwart eines Kupplungsreagenzes, wie beispielsweise 2-Ethoxy-1-ethoxycarbonyl-1,2-dihydrochinolin (EEDQ), 1-Isobutyloxycarbonyl 2-isobutyloxy-1,2-dihydrochinolin (IIDQ) oder Benzotriazol-1-yl-oxy-tris-(dimethylamino)-phosphonium-hexafluorphoshat (BOP), zu den Hydroxamsäuren der Formel III umgesetzt.
2) Die Verbindungen III werden dann durch Umsetzung mit den Verbindungen der Formel L¹-[CH(R³)]₂-L² cyclisiert [vgl. WO-A 95/04728].
   Die Reaktionsfolge II → III → IVa kann zweistufig oder vorzugsweise einstufig, d.h. ohne Isolierung der im ersten Verfahrensschritt gebildeten Hydroxamsäure III durchgeführt werden. Die Durchführung kann analog den in WO 95/04728, WO 97/00866 und EP-A 846 691 beschriebenen Methoden ausgeführt werden, auf die hiermit Bezug genommen wird.
3) Die Umwandlung der geschützten OH-Gruppe L^{a} in IVa in die reaktive Gruppe L in den Verbindungen IV ist aus der Literatur im Prinzip bekannt. Beispielsweise können Verbindungen IV, in denen L für Halogen steht, aus Verbindungen IVa durch Umsetzung mit einem Säurehalogenid, beispielsweise einem Carbonsäurechlorid wie Acetylchlorid, einem Sulfonsäurechlorid, oder einer Halogenwasserstoffsäure gegebenenfalls in Gegenwart einer Lewis-Säure, z.B. Aluminiumtrichlorid, hergestellt werden [vgl.: Jp-A 90/89653; DE-A 29 33 985; WO-A 95/04728; Tetrahedron Lett., Bd. 34, S. 3829 (1993)].
   Alternativ kann aus Verbindung IVa zunächst die Schutzgruppe abgespalten werden und anschließend die OH-Funktion im entstandenen Benzylalkohol-Derivat in eine reaktive Gruppe L übergeführt werden. [vgl. T. W. Greene, Protective Groups in Organic Chemistry, J. Wiley & Sons, 1991, S. 10 - 142]. Für L = Halogen gelingt dies beispielsweise durch Umsetzung mit üblichen Halogenierungsmitteln, wie SOCl₂, BBR₄/SnCl₄, CCl₄/Triphenylphosphin oder HBr [vgl.: Organikum, 16. Auflage 1988, S. 189ff., VEB Verlag der Wissenschaften, Berlin; Synthesis (1989) S. 614; J. Org. Chem., Bd. 53 (1988) S. 5113; Synth. Commun., Bd. 17 (1987) S. 219].

Verbindungen der Formel L¹-[CH(R³)]₂-L² sind literaturbekannt oder lassen sich nach bekannten Methoden herstellen [vgl.: WO-A 95/04728; WO-A 97/00866; EP-A 846 691].

Benzylverbindungen der Formel II sind ebenfalls literaturbekannt oder können nach bekannten Methoden hergestellt werden [vgl.: EP-A 178 826; EP-A 253 213; WO-A 95/04728].

Die Verbindungen der Formel IV mit L = Halogen, z.B. Chlor oder Brom, können alternativ auch aus Verbindungen der Formel IVa, in der L^{a} Wasserstoff bedeutet, durch Seitenkettenhalogenierung, z.B. mit N-Bromsuccinimid, hergestellt werden [vgl. WO 95/04728].

Die Umsetzung der Verbindung IV mit der Verbindung V bzw. VII (S. Schema 2, Schritte 1b bzw 2a) erfolgt beispielsweise in einem inerten Lösungs- oder Verdünnungsmittel, wie Aceton, Acetonitril, Dimethylsulfoxid, Dimethylformamid, N-Methylpyrrolidon etc., unter Verwendung einer Base (beispielsweise Natriumcarbonat, Kaliumcarbonat, Natriumhydroxid, Kaliumhydroxid, Natriumhydrid, Kaliumhydrid etc.). Die Basen werden im Allgemeinen äquimolar oder im Überschuss verwendet. Außerdem kann es vorteilhaft sein, eine katalytische Menge eines Kronen-ethers (beispielsweise 18-Krone-6 oder 15-Krone-5) zuzusetzen. Die Reaktionstemperatur liegt im Allgemeinen im Bereich von 0 bis 150 °C, vorzugsweise 20 bis 80°C.

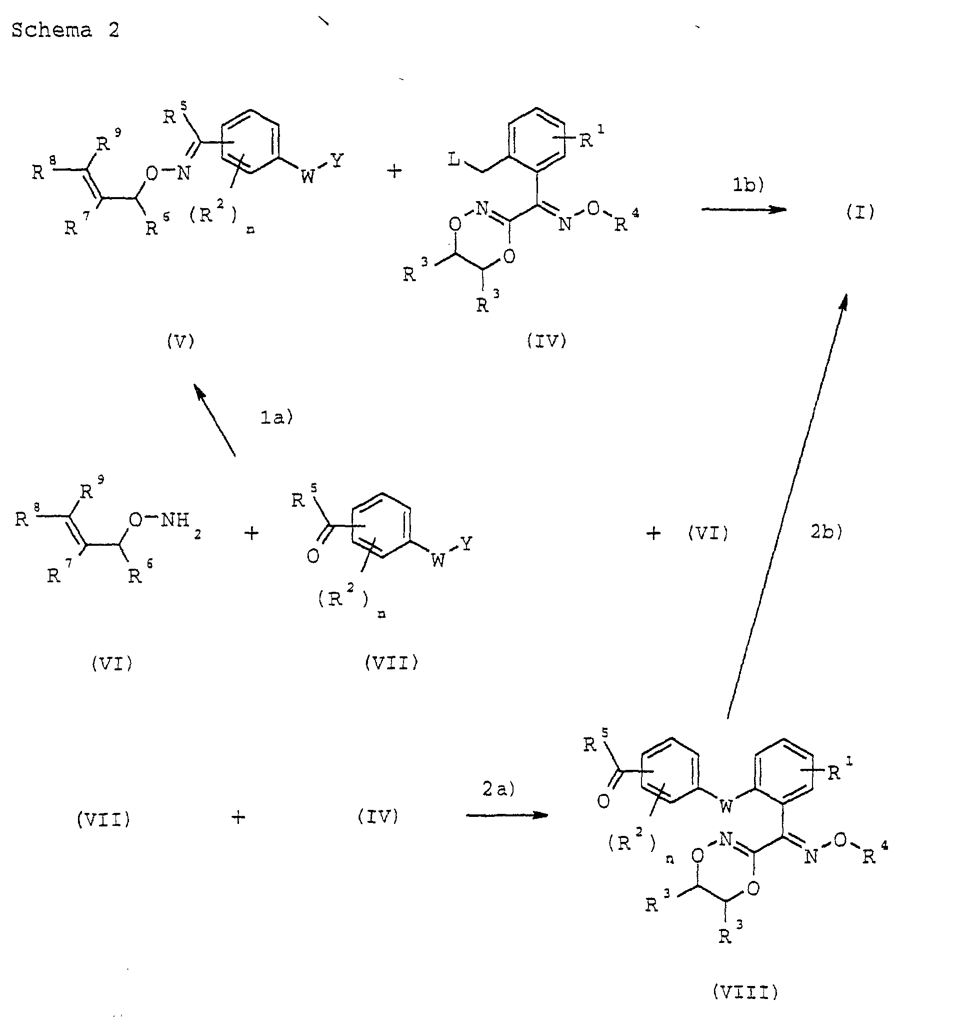

Die Herstellung der Verbindungen V bzw. VII ist dem Fachmann bekannt und beispielsweise beschrieben in Organikum, 17. Auflage, VEB Deutscher Verlag der Wissenschaften, Berlin 1988, S. 323ff, und Angewandte Chemie 1972, 84, 295 sowie Zh. Org. Khim 1995, 31, 601, Chem. Pharm. Bull. 1988, 36, 3134, Indian J. Chem., Sect. B, 1992, 31, 495. Bei der Gruppe W-Y handelt es sich um eine phenolische OH-Gruppe oder um die Gruppe C(R¹⁰)=N-OH. Die Gruppe L in Verbindung IV stellt - wie weiter oben ausgeführt - eine Abgangsgruppe dar, die mit dem Wasserstoffatom der phenolischen bzw. oximischen OH-Gruppe in Gegenwart einer Base abgespalten wird.

Die durch Umsetzung von IV mit VII erhaltene Verbindung VIII wird anschließend mit einem O-Alkenylhydroxylamin oder einem Salz davon oximiert (s. Schema 2, Schritte 2a und 2b). Die Herstellung des O-Alkenylhydroxylamins ist dem Fachmann bekannt und beispielsweise beschrieben in Chem. Pharm. Bull. 1983, 91, 2601 und J. Am. Chem. Soc. 1949, 71, 3423.

Alternativ kann eine Verbindung IV direkt mit einem Oxim V (s. Schema 2, Schritt 1b) zu einer Verbindung I umgesetzt werden. Alle Schritte des Weges 1), einschließlich der Herstellung der Zwischenprodukte bzw. analoger Zwischenprodukte, sind beschrieben in EP-A 386581 (für W = OCH₂) und in EP-A 585751 (für W = C(R¹⁰)=NOCH₂).

B) In Schema 3 wird eine alternative Syntheseroute zu den Verbindungen I dargestellt. Wie in Syntheseroute A) geht man von einer Verbindung II aus.

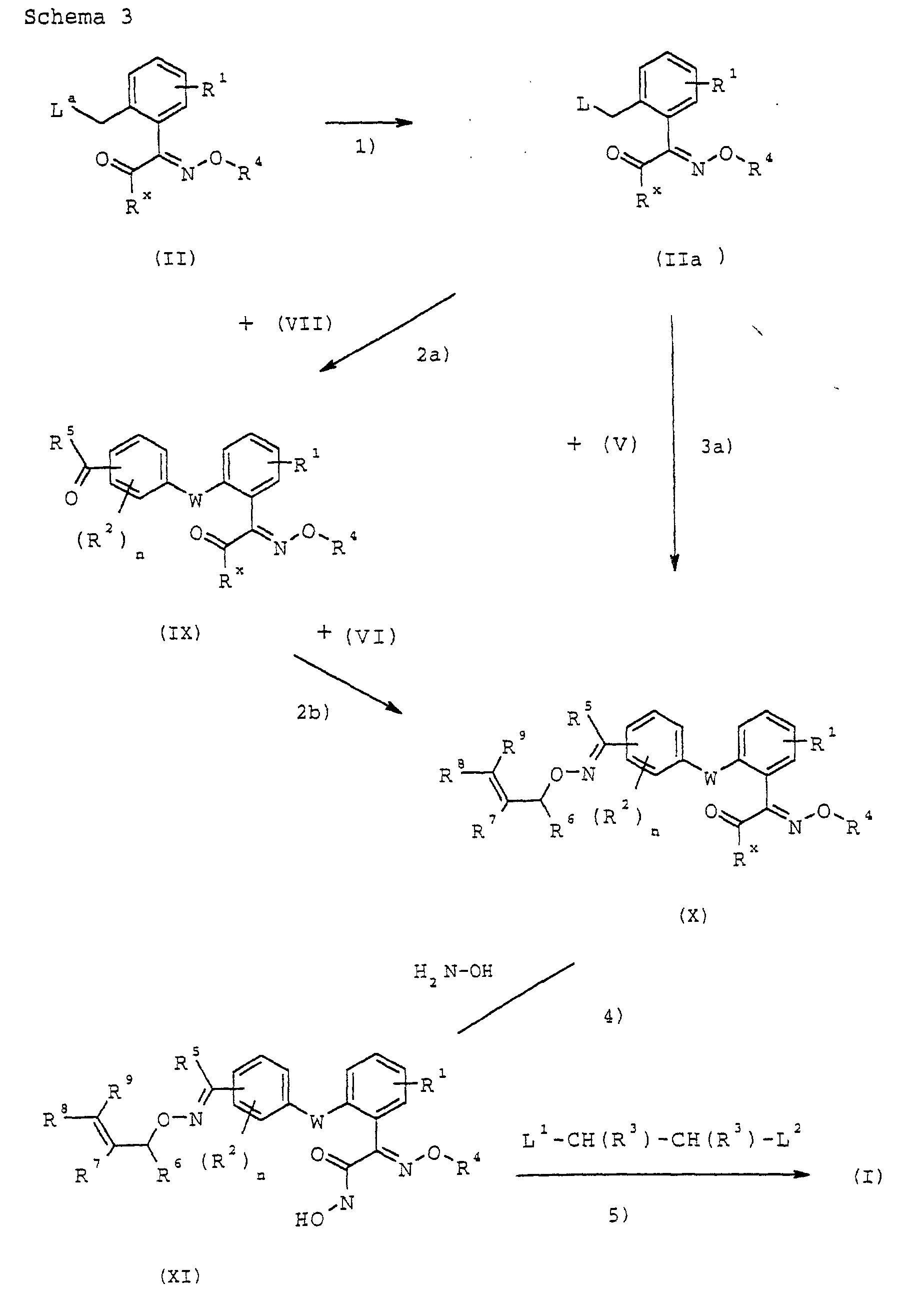

Die Umsetzung von IIa mit dem Oxim V (s. Schema 3, Schritt 3a) unter den oben genannten Veretherungsbedingungen (s. Schema 2, Schritt 1b) ergibt eine Verbindung X. Der Azadioxacycloalkenring wird dann in gleicher Weise wie beim Weg A) aufgebaut. Die Umsetzung mit Hydroxylamin führt zur Hydroxamsäure XI, die schließlich mit L¹-[CH(R³)]₂-L² zu den Verbindungen I ringgeschlossen wird.

Alternativ wird eine Verbindung IIa mit einer Verbindung VII unter den oben genannten Veretherungsbedingungen zu einer Verbindung IX umgesetzt. Diese wird, in gleicher Weise wie beim Weg A), mit O-Alkenylhydroxylamin (VI) oder einem Salz davon oximiert, wobei man die Verbindung X erhält, in die, wie schon beschrieben, anschließend der Azadioxacycloalkenring eingeführt wird.

Die erfindungsgemäßen Verbindungen können bei der Herstellung aufgrund der C=C- bzw. C=N-Doppelbindungen als E/Z-Isomerengemische anfallen. Diese können in üblicher Weise, beispielsweise durch Kristallisation oder Chromatographie, in die einzelnen Komponenten getrennt werden. Sowohl die einzelnen Isomerenverbindungen als auch ihre Gemische sowie alle Enantiomeren, Racemate und Diastereomeren werden von der Erfindung umfasst.

Die neuen Verbindungen I zeichnen sich durch eine hervorragende Wirksamkeit gegen ein breites Spektrum von pflanzenpathogenen Pilzen, insbesondere aus der Klasse der *Ascomyceten* und *Basidiomyceten*, aus und können als Blatt- und Bodenfungizide eingesetzt werden. Sie besitzen zum Teil bemerkenswert hohe systemische Beweglichkeit und Wirksamkeit nach Boden- und insbesondere auch nach Blattapplikation.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria*-Arten an Gemüse und Obst,
- *Botrytis cinerea* (Grauschimmel) an Erdbeeren, Gemüse, Zierpflanzen und Reben,
- *Cercospora arachidicola* an Erdnüssen,
- *Erysiphe cichoracearum* und *Sphaerotheca fuliginea* an Kürbisgewächsen,
- *Erysiphe graminis* (echter Mehltau) an Getreide,
- *Fusarium-* und *Verticillium*-Arten an verschiedenen Pflanzen,
- *Helminthosporium*-Arten an Getreide,
- *Mycosphaerella*-Arten an Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Rhizoctonia*-Arten an Baumwolle; Reis und Rasen,
- *Septoria nodorum* an Weizen,
- *Sphaerotheca fuliginea* (Gurkenmehltau) an Gurken,
- *Uncinula necator* an Reben,
- *Ustilago*-Arten an Getreide und Zuckerrohr, sowie
- *Venturia*-Arten (Schorf) an Äpfeln und Birnen.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nicht-ionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.
IX. 10 Gew.-Teile der erfindungsgemäßen Verbindung werden in 63 Gew.-Teilen Cyclohexanon, 27 Gew.-Teilen Dispergiermittel (beispielsweise eine Mischung aus 50 Gew.-Teilen des Anlagerungsprodukts von 7 Mol Ethylenoxid an 1 Mol Isooctylphenol und 50 Gew.-Teilen des Anlagerungsprodukts von 40 Mol Ethylenoxid an 1 Mol Ricinusöl) gelöst. Die Stammlösung wird anschließend durch Verteilen in Wasser auf die gewünschte Konzentration verdünnt, z.B. auf eine Konzentration im Bereich von 1 bis 100 ppm.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%. Häufig reichen bereits geringe Wirkstoffmengen an Verbindung I in der anwendungsfertigen Zubereitung aus, z.B. 2 bis 200 ppm. Ebenso sind anwendungsfertige Zubereitungen mit Wirkstoffkonzentrationen im Bereich von 0,01 bis 1 % bevorzugt.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis- (thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl- (4))-benzimidazol, N-(1,1,2,2-Tetrachlorethylthio) -tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1- [2-(2,4-Dichlorphenyl)-4-ethyl-1,3-dioxolan-2-yl-ethyl]-lH-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-ylharnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-l,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, a-(2-Chlorphenyl)-α-(4-chlorphenyl)-5-pyrimidin-methanol, 5-Butyl-2-dimethylamino-4-hydroxy-6-methyl-pyrimidin, Bis- (p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2- [6- (2-cyanophenoxy)-pyrimidin-4-yl-oxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid, Methyl-E-2-{2-[(2-trifluormethylpyridyl-6-)oxymethyl]-phenyl}-3-methoxyacrylat, (E,E)-Methoximino- {2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)-N-methoxycarbamat,
- Anilinopyrimidine wie N-(4,6-Dimethylpyrimidin-2-yl)-anilin, N- [4-Methyl-6-(1-propinyl) -pyrimidin-2-yl] -anilin, N- [4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl (2)-alaninat, DL-N- (2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N- (2,6-Dimethylphenyl)-N-(phenylacetyl)-alaninmethylester, 5-Methyl-5-vinyl-3-(3,5-dichlorphenyl)-2,4-dioxo-1,3-oxazolidin, 3- [3,5-Dichlorphenyl (-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano- [N-(ethylaminocarbonyl)-2-methoximino]-acetamid, 1- [2-(2,4-Dichlorphenyl)-pentyl]-1H-1,2,4-triazol, 2,4-Difluor-α-(1H-l,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl) -methyl)-1H-1,2,4-triazol.

### Synthese der Vorstufen

### Synthese von 1,4-Diacetylbenzolmonoxim

In 80 ml Methanol wurden 25 g (0,154 mol) 1,4-Diacetylbenzol und 12,2 g (0,154 mol) Pyridin gelöst. Unter Rückfluss wurde langsam eine Lösung von 10,7 g (0,154 mol) Hydroxylammoniumchlorid in 50 ml Wasser zugegeben. Es wurde 6 h unter Rückfluss und 15 h bei Raumtemperatur gerührt. Anschließend filtrierte man, wusch den Rückstand mit Wasser und trocknete ihn im Vakuum. Man erhielt 24,7 g (90 %) des Produktes als farblose Kristalle. Schmelzpunkt: 162 - 169 °C.

Synthese von 3-[α-Methoximino-α-(2'-chloromethylphenyl)methyl]-5,6- dihydro-1,4,2-dioxazin

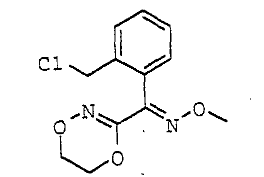

Zu einer Suspension von 5,2 g Aluminiumtrichlorid in 50 ml wasserfreiem Dichlormethan wurden 3,66 g Acetylchlorid getropft. Anschließend wurde eine Lösung von 5,3 g α-Methoximino-[ortho-(2-methylphenoxymethylen)-phenyl]essigsäuremethylester in 20 ml wasserfreiem Dichlormethan zugetropft und 3 Std. bei 20 bis 25 °C gerührt. Nach Verdünnen mit 100 ml Dichlormethan wurde die Reaktionslösung auf Eiswasser gegossen. Nach Phasentrennung wurde die organische Phase mit Wasser gewaschen, getrocknet und vom Lösungsmittel befreit. Nach Chromatographie an Kieselgel (Cyclohexan-Ethylacetat-Gemisch [1:1]) erhielt man 3,14 g der Titelverbindung als hellbeige Kristalle vom Fp. 93-96 °C.

### Synthesebeispiele

### Beispiel 1

### Herstellung von (1) (Verbindung I.1, Tabelle B)

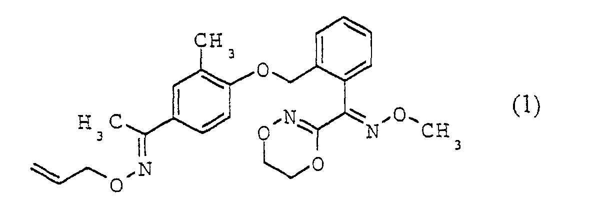

### 1.1 Herstellung der Zwischenverbindung (1.1)

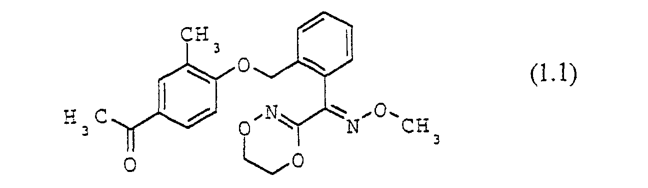

Zu einer Lösung von 3,0 g (20 mmol) *p*-Hydroxy-*m*-methyl-acetophenon in 75 ml wasserfreiem DMF wurden 0,53 g (22 mmol) Natriumhydrid gegeben. Nachdem 1 h bei Raumtemperatur gerührt wurde, gab man 5,47 g (20 mmol) 3-[α-Methoximino-α-(2'-chloromethylphenyl)methyl]-5,6- dihydro-1,4,2-dioxazin (s. obige Vorschrift) zu, rührte 4 h bei 90°C und 18 h bei Raumtemperatur. Der Ansatz wurde mit 1 l verd. NaCl-Lösung aufgenommen und mit Methyltert.-butylether extrahiert. Die vereinigten organischen Phasen wurden mit Wasser gewaschen, über Natriumsulfat getrocknet und im Vakuum eingeengt. Nach *flash*-chromatographischer Reinigung erhielt man 6,0 g der Titelverbindung als hellgelben, amorphen Feststoff mit Fp. = 144-145°C.

### 1.2 Herstellung von (1)

Eine Lösung von 1,91 g (5 mmol) von Verbindung 1.1, 0,82 ( 7.5 mmol) O-Propenhydroxylaminhydrochlorid und 0,59 g (7.5 mmol) Pyridin in 20 ml Methanol wurde 18 h bei Raumtemperatur gerührt. Anschließend wurde im Vakuum eingeengt und der Rückstand in 150 ml Dichlormethan aufgenommen. Es wurde mit verd. HCl-Lösung und Wasser gewaschen, über Natriumsulfat getrocknet und im Vakuum eingeengt. Man erhielt 1,92 g der Titelverbindung mit Fp = 112-116°C.

### Beispiel 2 (Tabelle C, Beispiel I.16)

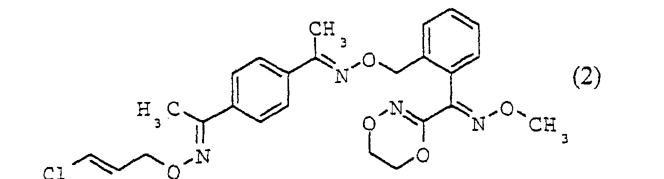

### 1.1 Herstellung der Zwischenverbindung (2.1)

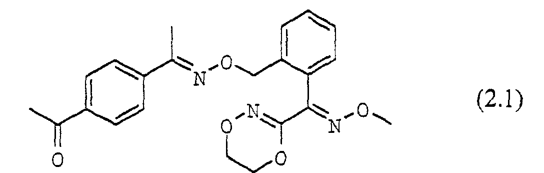

In 6 ml wasserfreiem DMF wurden 0,53 g Natriumhydrid suspendiert. Man tropfte eine Lösung von 5,45 g 1,4-Diacetylbenzolmonoxim in 34 ml wasserfreiem DMF zu, rührte 10 min im Ultraschallbad und 1 h bei Raumtemperatur. Anschließend wurden 5,37 g 3-[α-Methoximino-α-(2'-chloromethylphenyl)methyl]-5,6-dihydro-1,4,2-dioxazin (s. obige Vorschrift) zugegeben und es wurde 4 h bei 90°C und 16 h bei Raumtemperatur gerührt. Es werden 500 ml verd. NaCl-Lösung zugegeben. Man extrahierte dreimal mit Methyltert.-butylether. Die vereinigten organischen Phasen wurden mit Wasser gewaschen, über Natriumsulfat getrocknet und im Vakuum eingeengt. Der Rückstand wurde mit Methanol verrührt, filtriert und mit n-Pentan gewaschen. Man erhielt 2,9 g (41 %) der Titelverbindung als hellbeige Kristalle mit Fp. = 154-158°C.

### Synthese von 2

Zu einer Lösung von 0,65 g der Vorstufe 2,1 in 10 ml Methanol wurden 0,35 g 3-Chlorallylhydroxylaminhydrochlorid und 0,18 g wasserfreies Pyridin gegeben. Man rührte das Gemisch 16 h. Anschließend wurde im Vakuum eingeengt und der Rückstand mit Dichlormethan aufgenommen. Es wurde mit 2%iger Salzsäure und Wasser gewaschen, über Natriumsulfat getrocknet und im Vakuum eingeengt. Man erhielt 0,69 g (64 %) des Produktes als farbloses Öl (phys. Daten vgl. Tabelle C, Beispiel I.16)).

Die übrigen in den nachfolgenden Tabellen B und C aufgeführten Verbindungen wurden in analoger Weise erhalten.

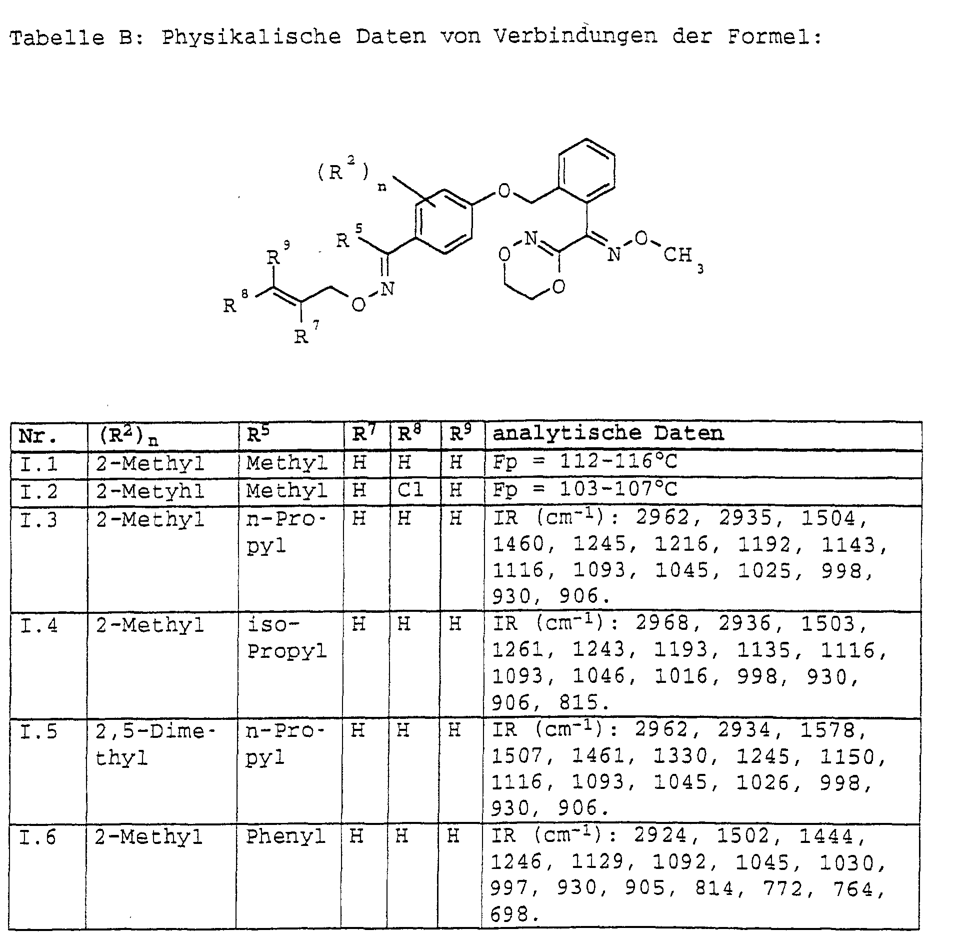

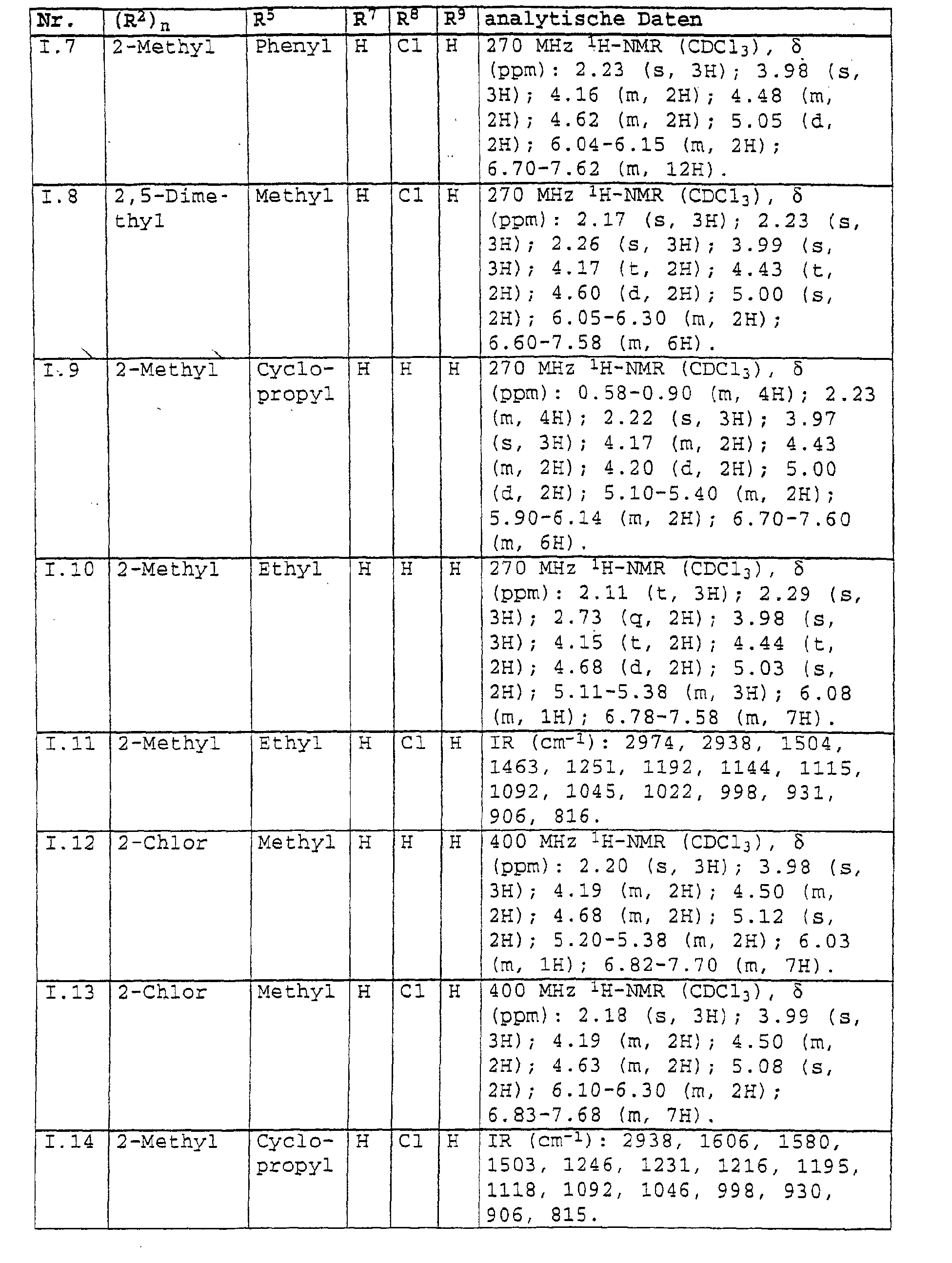

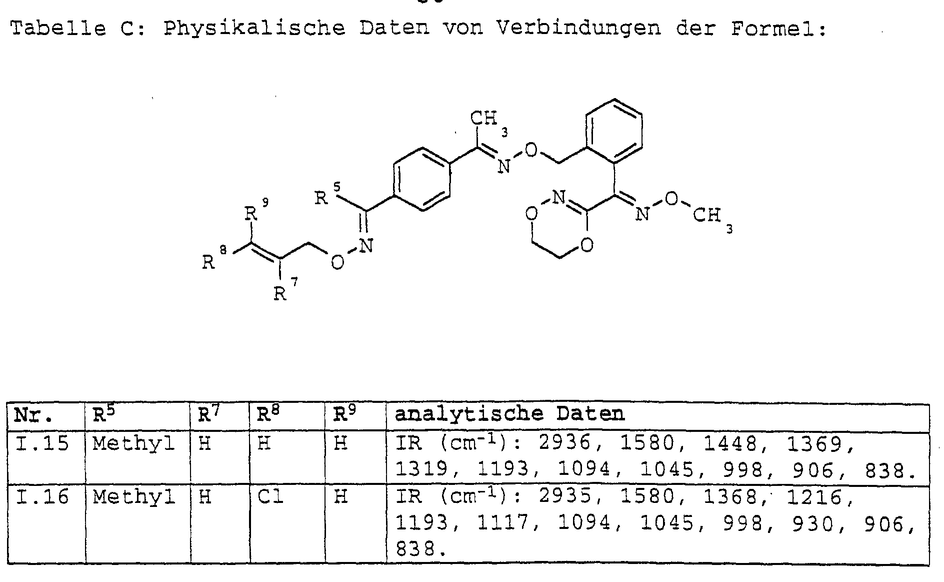

### Anwendungsbeispiele

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Azadioxacycloalkene der Formel I 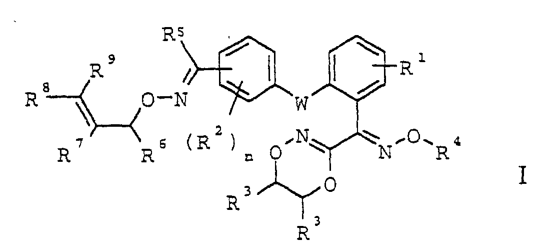 in der die Substituenten die folgenden Bedeutungen haben:
W -OCH₂-, -C(R¹⁰)=N-O-CH₂- ;
R¹ Wasserstoff, C₁-C₄-Alkyl, Halogen, Nitro, CN, C₁-C₄-Halogenalkyl, C₁-C₄-Alkoxy;
R² Wasserstoff, C₁-C₄-Alkyl, Halogen, Nitro, CN, C₁-C₄-Halogenalkyl, C₁-C₄-Alkoxy;
n 1 oder 2;
R³ Wasserstoff, Methyl oder Ethyl;
R⁴ C₁-C₄-Alkyl;
R⁵ Wasserstoff, C₁-C₆-Alkyl, C₂-C₄-Alkenyl, C₁-C₄-Halogenalkyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl, C₃-C₆-Cycloalkyl;
R⁶ Wasserstoff, C₁-C₄-Alkyl, C₁-C₄-Halogenalkyl, C₂-C₄-Alkenyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl;
R⁷ Wasserstoff, Halogen, C₁-C₆-Alkyl, C₁-C₄-Halogenalkyl, C₂-C₆-Alkenyl, C₂-C₆-Halogenalkenyl, C₃-C₆-Cycloalkyl, C₃-C₆-Halogencycloalkyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl;
R⁸ Wasserstoff, Halogen, C₁-C₆-Alkyl, C₁-C₆-Halogenalkyl, C₂-C₆-Alkenyl, C₂-C₆-Halogenalkenyl, C₃-C₆-Cycloalkyl, C₃-C₆-Halogencycloalkyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl, oder
R⁷ und R⁸ bilden, zusammen mit den Kohlenstoffatomen, an die sie gebunden sind, einen ungesättigten Heterocyclus mit 5- oder 6-Ringatomen, der ein oder zwei Heteroatome aufweist, die unabhängig voneinander ausgewählt sind unter einem Stickstoff-, Sauerstoff- und Schwefelatom und der gegebenenfalls mit einem oder zwei Resten substituiert sein kann, die unabhängig voneinander ausgewählt sind unter C₁-C₄-Alkyl, Halogen, Nitro, CN, C₁-C₄-Halogenalkyl, C₁-C₄-Alkoxy, C₂-C₄-Alkenyl, C₂-C₄-Halogenalkenyl, C₂-C₄-Alkinyl, C₂-C₄-Halogenalkinyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl;
R⁹ Wasserstoff, Halogen, C₁-C₆-Alkyl, C₁-C₆-Halogenalkyl, C₂-C₆-Alkenyl, C₂-C₆-Halogenalkenyl, C₃-C₆-Cycloalkyl, C₃-C₆-Halogencycloalkyl, unsubstituiertes oder durch Halogen, Nitro, CN, C₁-C₄-Alkyl, C₁-C₄-Halogenalkyl, C₁-C₄-Alkoxy oder C₁-C₄-Halogenalkoxy substituiertes Phenyl;
R¹⁰ Wasserstoff, Halogen, C₁-C₄-Alkyl.

2. Verbindungen der Formel I nach Anspruch 1, wobei die Substituenten die folgenden Bedeutungen haben:
R¹ Wasserstoff, C₁-C₄-Alkyl, Halogen, C₁-C₄-Halogenalkyl;
R² Wasserstoff, C₁-C₄-Alkyl, Halogen, C₁-C₄-Halogenalkyl;
R⁴ Methyl oder Ethyl;
R⁵ Wasserstoff oder C₁-C₄-Alkyl;
R⁶ Wasserstoff, C₁-C₄-Alkyl, C₁-C₄-Halogenalkyl;
R⁷ Wasserstoff, Halogen, C₁-C₆-Alkyl, C₁-C₆-Halogenalkyl, C₃-C₆-Cycloalkyl, C₃-C₆-Halogencycloalkyl, Phenyl;
R⁸ Wasserstoff, Halogen, C₁-C₆-Alkyl, C₁-C₆-Halogenalkyl, C₃-C₆-Cycloalkyl, C₃-C₆-Halogencycloalkyl, C₂-C₆-Alkenyl, Phenyl, das durch ein oder zwei Halogen oder C₁-C₄-Alkyl sustituiert sein kann; oder
R⁷ und R⁸ zusammen mit den Kohlenstoffatomen, an die sie gebunden sind, bilden einen ungesättigten Heterocyclus mit 5- oder 6-Ringatomen, der ein oder zwei Heteroatome aufweist, die unabhängig voneinander ausgewählt sind unter einem Stickstoff-, Sauerstoff- und Schwefelatom und der gegebenenfalls mit einem oder zwei Resten substituiert sein kann, die unabhängig voneinander ausgewählt sind unter C₁-C₄-Alkyl, Halogen, C₁-C₄-Halogenalkyl, C₁-C₄-Alkoxy und Phenyl, das durch ein oder zwei Halogen oder C₁-C₄-Alkyl substituiert sein kann;
R⁹ Wasserstoff, Halogen, C₁-C₆-Alkyl, C₁-C₆-Halogenalkyl, C₃-C₆-Cycloalkyl, C₃-C₆-Halogencycloalkyl, Phenyl;

3. Verbindungen der Formel I nach Anspruch 1 oder 2, wobei die Substituenten die folgenden Bedeutungen haben:
R¹ Wasserstoff, Methyl, Halogen;
R² Wasserstoff, C₁-C₄-Alkyl, Halogen;
R³ Wasserstoff;
R⁶ Wasserstoff, C₁-C₄-Alkyl;
R⁷ Wasserstoff, Halogen, C₁-C₆-Alkyl;
R⁸ Wasserstoff, Halogen, C₁-C₆-Alkyl, C₂-C₆-Alkenyl; oder
R⁷ und R⁸ bilden zusammen mit den Kohlenstoffatomen, an die sie gebunden sind, Thienyl, Furanyl, Oxazolyl, Thiazolyl, wobei diese Gruppen ein oder zwei Substituenten aufweisen können, die unabhängig voneinander ausgewählt sind unter C₁-C₄-Alkyl, Halogen und Phenyl, das durch ein oder zwei Halogen substituiert sein kann;
R⁹ Wasserstoff, Halogen, C₁-C₆-Alkyl;
R¹⁰ Wasserstoff, C₁-C₄-Alkyl;

4. Verbindungen der Formel I nach einem der vorhergehenden Ansprüche, wobei die Substituenten die folgenden Bedeutungen haben:
R¹ Wasserstoff;
R⁷ Wasserstoff, Halogen;
R⁸ Wasserstoff, C₁-C₄-Alkyl, Halogen; oder
R⁷ und R⁸ bilden zusammen mit den Kohlenstoffatomen, an die sie gebunden sind, Thienyl oder Oxazolyl, wobei diese Gruppen gegebenenfalls durch ein oder zwei Halogen oder Phenyl substituiert sind und das Phenyl durch ein oder zwei Halogen substituiert sein kann;
R⁹ Wasserstoff, Halogen;
R¹⁰ Wasserstoff, C₁-C₄-Alkyl.

5. Verwendung der Verbindungen der Formel I nach einem der Ansprüche 1 bis 4 als Fungizide oder zur Bekämpfung von tierischen Schädlingen.

6. Fungizides Mittel, enthaltend feste und/oder flüssige Trägerstoffe und eine fungizid wirksame Menge wenigstens einer Verbindung der Formel I gemäß Anspruch 1.

7. Verfahren zur Bekämpfung von pflanzenpathogenen Pilzen, wobei man die Pilze oder die von Pilzbefall bedrohten Materialien, Pflanzen, Saatgüter oder den Erdboden mit einer fungizid wirksamen Menge mindestens einer Verbindung der Formel I gemäß Anspruch 1 behandelt.

8. Mittel zur Bekämpfung von tierischen Schädlingen, enthaltend inerte Zusatzstoffe und eine pestizid wirksame Menge mindestens einer Verbindung der Formel I gemäß Anspruch 1.

9. Verfahren zur Bekämpfung von tierischen Schädlingen, wobei man die Schädlinge und/oder deren Lebensraum mit einer pestizid wirksamen Menge mindestens einer Verbindung der Formel I gemäß Anspruch 1 behandelt.
